# EUROPÄISCHE PATENTSCHRIFT

(11) **EP 0 662 951 B1**
(45) Veröffentlichungstag und Bekanntmachung des Hinweises auf die Patenterteilung: **30.12.1998**
(21) Anmeldenummer: 93921893.9
(22) Anmeldetag: 30.09.1993
(51) Int. Cl.: C07C 315/00, C07C 317/44, C07D 263/26

(54) **VERFAHREN ZUR STEREOSELEKTIVEN SYNTHESE VON 3-SUBSTITUIERTEN 2-THIOMETHYLPROPIONSÄUREN**
PROCESS FOR THE STEREOSELECTIVE SYNTHESIS OF 3-SUBSTITUTED 2-THIOMETHYL PROPIONIC ACIDS
PROCEDE DE SYNTHESE STEREOSELECTIVE D'ACIDES 2-THIOMETHYLPROPIONIQUES SUBSTITUES EN POSITION 3

(30) Priorität: 01.10.1992 DE 4233099
(43) Veröffentlichungstag der Anmeldung: 19.07.1995
(73) Patentinhaber: HOECHST AKTIENGESELLSCHAFT, 65929 Frankfurt am Main (DE)
(72) Erfinder: HOLLA, Wolfgang, D-65830 Kriftel am Taunus (DE); KAMMERMEIER, Bernhard, D-60529 Frankfurt am Main (DE); BECK, Gerhard, D-60320 Frankfurt am Main (DE)
(86) Internationale Anmeldenummer: EP9302673
(87) Internationale Veröffentlichungsnummer: WO9407849

(56) Entgegenhaltungen:
- EP-A- 0 309 766
- EP-A- 0 332 008
- JOURNAL OF ORGANIC CHEMISTRY, Bd. 50, Nr. 11 , 31. Mai 1985 Washington, DC, US, Seiten 1830 - 1835 D.A. EVANS, ET AL.: 'Asymetric synthesis of the encephalinase inhibitor Thiorphan' in der Anmeldung erwähnt
- JOURNAL OF MEDICINAL CHEMISTRY, Bd. 31, Nr. 9 , September 1988 Washington, DC, US, Seiten 1839 - 1846 P. BÜHLMAYER, ET AL.: 'Synthesis and biological activity of some transition-state inhibitors of human renin' in der Anmeldung erwähnt

## Beschreibung

3-Substituierte 2-Thiomethylpropionsäüren und deren Derivate, z. B. Ester verdienen Interesse als Bausteine sowie als Vorstufen für Aspartylprotease-Inhibitoren [P. Bühlmeyer et al., J. Med. Chem. 31 (1988), 1839; R. Henning, Nachr. Chem. Techn. Lab. 38 (1990), 460; J. R. Huff, J. Med. Chem. 34 (1991), 2305].

Die genannten Verbindungen sind bereits bekannt. Es ist weiterhin eine Reihe von Synthesen bzw. Verfahren zur Herstellung dieser Verbindungen beschrieben.

Stellvertretend seien hier einige aktuelle Arbeiten genannt:

P. Bühlmeyer et al., J. Med. Chem. 31 (1988), 1839; K. Tsuji et al., Tetrahedron Lett. 30 (1989), 6189; M. Nakano et al., Tetrahedron Lett. 31 (1990), 1569; M. Nakano et al., Chem. Lett. (1990), 505; vgl. auch D. A. Evans et al., J. Org. Chem. 50 (1985), 1830.

Die erstgenannte Synthese erfordert unter anderem die Verwendung von Formaldehyd bzw. Formaldehyd-Derivaten, die ebenso wie dabei entstehende Nebenprodukte aufgrund ihrer alkylierenden Eigenschaften als gesundheitlich äußerst bedenklich gelten und spezielle, auch arbeitshygienische Sicherheitsvorkehrungen [Merck-lndex 11, 4150] erfordern.

Darüber hinaus ist zur Herstellung optisch reiner Verbindungen eine Racematspaltung erforderlich. Dazu wird die racemische Säure mit L-Phenylalaninol in die diastereomeren Amide überführt, diese chromatographisch getrennt und das gewünschte Isomere durch Hydrolyse des entsprechenden Amids erhalten. Die Vielzahl der Stufen, die grundsätzlichen Nachteile einer Racematspaltung und die Probleme, die eine chromatographische Reinigung beim scale up verursacht, lassen diese Route für die Herstellung größerer Mengen nicht attraktiv erscheinen.

Die Synthese nach Tsuyi et al. führt bei ähnlicher Stufenzahl, jedoch ohne Racematspaltung, zu optisch reinem Material. Diese Synthese umfaßt eine Reihe von Schritten zur Einführung und Abspaltung von Schutzgruppen; es werden zum Teil teure Reagenzen eingesetzt, karzinogene Formaldehyd-Derivate verwendet bzw. im Laufe der Synthesen hergestellt [H. G. Neumann in "Allgemeine und spezielle Pharmakologie und Toxikologie", 4. Auflage, W. Forth, Hrsg., B. I. Wissenschaftsverlag, Mannheim-Wien-Zürich, S. 621 ff (1983); Arch. Environ. Health 30 (2), 61].
Für die Darstellung großer Mengen stellt dieser Syntheseweg somit keine ökonomisch und ökologisch vertretbare Alternative dar.

Die im Rahmen der von Evans und Mathre beschriebenen Thiorphan-Synthese geschilderte Darstellung von 2-Mercaptomethyldihydrozimtsäure ist im Vergleich zu den beiden bereits diskutierten Synthesen kurz, verläuft mit guter Gesamtausbeute und ermöglicht die gezielte Darstellung beider Enantiomerer in hoher optischer Reinheit. Schwachpunkt dieser Synthese ist die umständliche Einführung der Mercaptangruppierung und die dazu nötige Verwendung des gesundheitlich bedenklichen Benzylthiomethylbromids, welches aus Trioxan (als Formaldehyd-Quelle), Benzylmercaptan (bzw. Benzylthiomethylchlorid) und HBr hergestellt wird [H. G. Neumann in "Allgemeine und spezielle Pharmakologie und Toxikologie", 4. Auflage, W. Forth, Hrsg., B. I. Wissenschaftsverlag, Mannheim-Wien-Zürich, S. 621 ff (1983); Arch. Environ. Health 30 (2), 61].

Nakano et al. beschreiben zwei Routen zum Aufbau des Kohlenstoffgerüstes der Verbindungen I. Die erste Synthese geht aus von Malonsäurediethylester, erfordert sechs Stufen und zusätzlich eine chromatographische Diastereomeren-Trennung.

Die zweite Route verläuft über ein chirales, nicht-racemisches Arylpropionyloxazolidinon und dessen stereoselektiver Alkylierung mit Benzylbrommethylether [M. W. Holladay et al., J. Med. Chem. 30 (1987), 374] und anschließender Chromatographie. Der Schwefelsubstituent wird nach Entfernung der Benzylgruppe durch Hydrierung - wie bei der ersten Route - durch Tosylierung der freien Hydroxygruppe und anschließender Substitution mit NaSCH₂CH₃ in DMF eingeführt.

Auch diese beiden Synthesewege sind gekennzeichnet durch: hohe Stufenzahl, karzinogene bzw. toxische Formaldehyd-Derivate als Vorstufen bzw. Reagenzien, die umständliche und nicht ganz racemisierungsfreie Einführung des Schwefelsubstituenten und nicht zuletzt die chromatographische Abtrennung von Verunreinigungen und diastereomeren Verbindungen.

Ein weiteres allgemeines Verfahren zur Herstellung von substituierten Thioalkylcarbonsäuren ist in dem Dokument EP 0 309 766 beschrieben; das genannte Verfahren unterscheidet sich allerdings von dem Verfahren gemäß der vorliegenden Patentanmeldung durch die Wahl der Ausgangsverbindungen und durch die Anzahl und Reihenfolge der Reaktionsschritte.

Der vorliegenden Erfindung liegt die Aufgabe zugrunde ein Verfahren zur Synthese von Verbindungen der Formel I zu entwickeln, das
* eine geringe Stufenzahl aufweist,
* keine Schutzgruppenchemie erfordert,
* keine säulenchromatographischen Schritte erfordert,
* stereoselektiv ist und wahlweise zu den enantiomeren Verbindungen der Formel I führt,
* keine Racematspaltung oder Diastereomerentrennung erfordert und
* unter gesundheitlichen, ökologischen und sicherheitstechnischen Gesichtspunkten eine Verbesserung darstellt bzw. unbedenklich ist.

Diese Aufgabe wird durch das erfindungsgemäße Verfahren gelöst. Erfindungsgegenstand ist demzufolge ein Verfahren zur stereoselektiven Herstellung einer Verbindung der Formel I 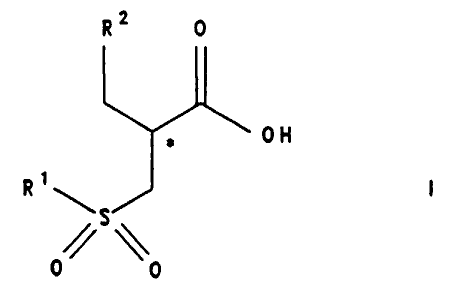 worin
R¹ C₁-C₆-Alkyl, C₃-C₆-Cycloalkyl, (C₆-C₁₂-Aryl-(C₁-C₄)-alkyl oder C₆-C₁₂-Aryl bzw. -Heteroaryl bzw. -Heterocycloalkyl, die mit 1, 2 oder 3 gleichen oder verschiedenen Hydroxy-, Methoxy- oder Trialkylsilyloxygruppen substituiert sein können; und
R² C₆-C₁₂-Aryl, das mit 1, 2 oder 3 gleichen oder verschiedenen Methoxy-, Halogen-, Cyano-, Methyl-, Trifluormethyl-, Isopropyl- oder Nitrogruppen substituiert sein kann;
C₃-C₉-Heteroaryl, das mit 1, 2 oder 3 gleichen oder verschiedenen Methoxy-, Halogen-, Cyano-, Methyl-, Trifluormethyl, Isopropyl- oder Nitrogruppen substituiert sein kann; oder
C₁-C₁₀-Alkyl, Alkenyl oder Alkinyl, die durch 1, 2 oder 3 gleiche oder verschiedene Methoxy-, Halogen-, Cyano-, Methyl-, Trifluormethyl, Isopropyl- oder Nitrogruppen substituiert sein können, bedeuten
und die Verbindungen der Formel I in der R- oder S-Form vorliegen können, dadurch gekennzeichnet, daß man eine Verbindung der Formel lla oder llb bzw. IIIa oder IIIb (chirales Auxiliar), worin Y = O oder S ist, 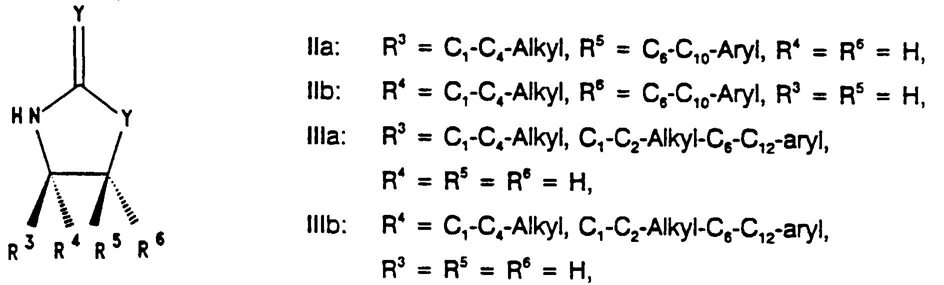
a₁) mit Acrylsäure oder mit Derivaten der Acrylsäure in eine Verbindung der Formel IVa oder IVb bzw. Va oder Vb, worin Y = O oder S ist, 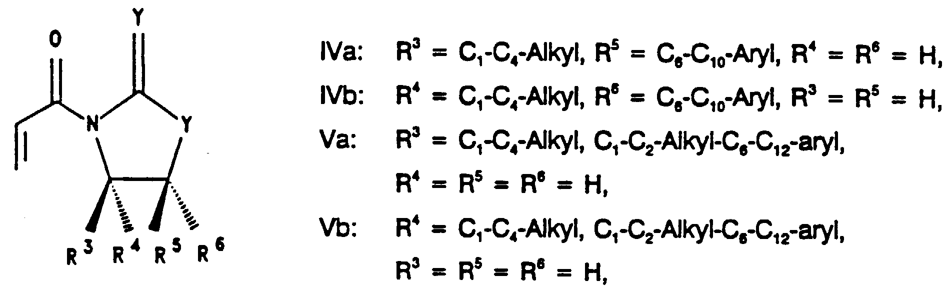 überführt, oder
a₂) mit Propionsäure oder Propionsäurederivaten, die ihrerseits in C₃-Position durch einen Rest X = Cl, Br, OTs, OMs substituiert sind, in eine Verbindung der Formel VIa oder VIb bzw. VIIa oder VIIb, worin X und Y die obengenannte Bedeutung haben, 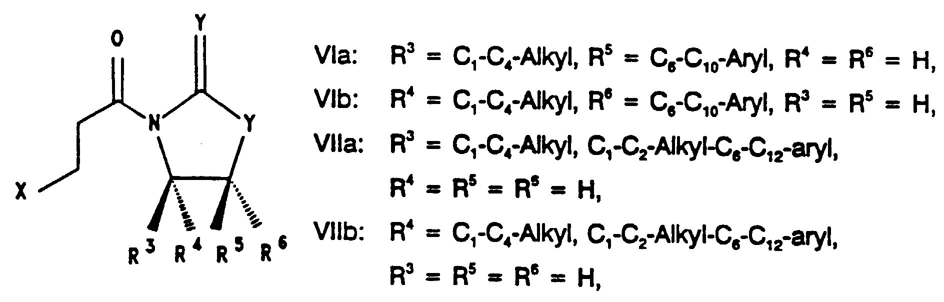 überführt,
b) die nach a₁) oder a₂) erhaltene Verbindung der Formel IVa, IVb, Va, Vb, VIa, VIb, VIIa oder VIIb direkt oder nach Isolierung in nichtwäßrigem Milieu oder in Gegenwart von Phasentranster-Katalysatoren in wäßrigen Zweiphasen-Systemen mit einem Mercaptan der Formel R¹SH, worin R¹ wie oben definiert ist, in die entsprechende Verbindung der Formel VIIIa oder VIIIb bzw. IXa oder IXb,
   worin R¹ und Y die obengenannte Bedeutung haben, 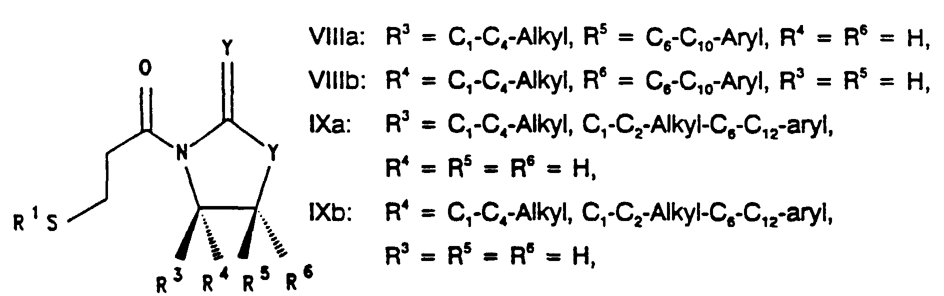 umsetzt, oder
a₃) eine Verbindung der Formel IIa oder IIb bzw. IIIa oder IIIb in Gegenwart einer für die Deprotonierung des Oxazolidinons sowie des Mercaptans R¹SH, worin R¹ obengenannte Bedeutung hat, ausreichenden Menge Base durch Umsetzung mit Acryl- oder Propionsäurehalogeniden oder deren jeweiligen gemischten Anhydriden und weiterer Umsetzung des Reaktionsproduktes mit dem Mercaptan in einem Schritt in eine Verbindung der Formel Vllla oder Vlllb bzw. IXa oder IXb überführt,
c) die nach b) oder a₃) erhaltene Verbindung anschließend durch stereoselektive Alkylierung in die entsprechende Verbindung der Formel Xa, Xb, Xla oder Xlb überführt, 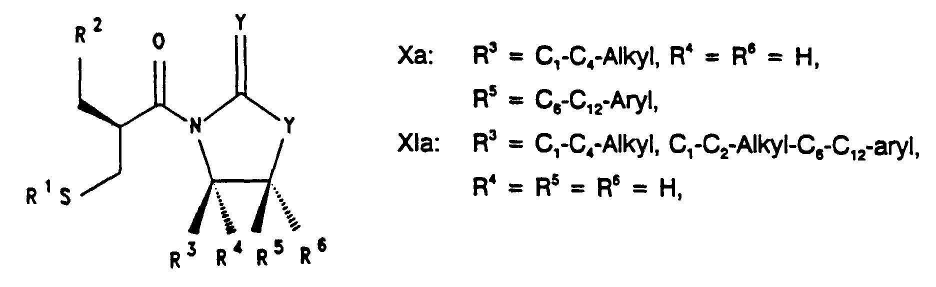 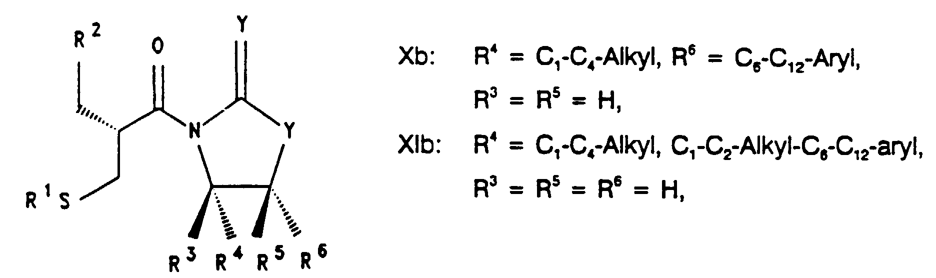 worin Y, R¹ und R² die obengenannte Bedeutung haben,
d) die derart gewonnene Verbindung dann durch Umsetzung mit H₂O₂, in alkalischer Lösung, bevorzugt mit LiOH/H₂O₂, in Verbindungen der Formel XII, die in der R- oder S-Form vorliegen können, 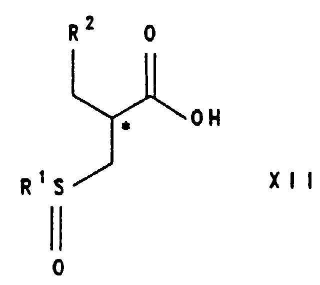 überführt und
e) die so erhaltene Verbindung dann durch weitere Oxidation in die entsprechende Verbindung der Formel I überführt.

Das erfindungsgemäße Verfahren eignet sich besonders zur Herstellung von Verbindungen der Formel I, in der
R¹ C₁-C₄-Alkyl, C₅-C₆-Cycloalkyl, (C₆-C₁₂)-Aryl-(C₁-C₃)-alkyl oder C₆-C₁₂-Aryl bzw. -Heteroaryl bzw. -Heterocycloalkyl, die mit einer Hydroxy-, Methoxy- oder Trialkylsilyloxygruppe substituiert sein können;
R² C₆-C₁₂-Aryl, das mit einer Methoxy-, Halogen-, Methyl-, Trifluormethyl-, oder Isopropylgruppe substituiert sein kann;
C₃-C₆-Heteroaryl, das mit einer Methoxy-, Halogen-, Methyl-, Trifluormethyl, oder Isopropylgruppe substituiert sein kann oder C₁-C₆-Alkyl, -Alkenyl oder -Alkinyl, bedeuten.

Weiterhin eignet sich das erfindungsgemäße Verfahren besonders zur Herstellung von Verbindungen der Formel I, in der
R¹ C₁-C₄-Alkyl,
(C₆-C₁₂)-Aryl-(C₁-C₃)-alkyl oder C₆-C₁₂-Aryl und
R² = C₆-C₁₂-Aryl, C₃-C₆-Heteroaryl oder C₁-C₄-Alkyl, -Alkenyl oder -Alkinyl bedeuten.

Eine ganz besondere Bedeutung besitzt das erfindungsgemäße Verfahren zur Herstellung von Verbindungen der Formel I, in der R¹ = tert.-Butyl und R² = Naphthyl bedeuten.

Unter dem vorstehend genannten Substituenten C₆-C₁₂-Aryl wird beispielsweise Phenyl, Naphthyl oder Biphenyl verstanden.

Alkyl, Alkenyl und Alkinyl können geradkettig oder verzweigt sein.

Halogen steht vorzugsweise für Fluor, Chlor oder Brom, besonders bevorzugt für Chlor.

Heterocycloalkyl bzw. Heteroaryl steht insbesondere für Verbindungen, die bis zu 3, vorzugsweise 2, insbesondere 1 N, S und/oder O Atom im Ring enthalten. Beispielshaft seien genannt Pyridyl, Furyl, Pyrimidyl, Pyrrolidino, Pyrolyl, Piperidino oder Piperidyl, insbesondere Pyridyl.

Bevorzugt verwendete Verbindungen der Formeln IIa, IIb, IIIa und IIIb sind solche worin,
- IIa:: R³ = Me, R⁵ = Ph, R⁴ = R⁶ = H
- IIb:: R⁴ = Me, R⁶ = Ph, R³ = R⁵ = H
- IIIa:: R³ = CH(CH₃)₂, R⁴ = R⁵ = R⁶ = H
- IIIb:: R⁴ = CH(CH₃)₂, R³ = R⁵ = R⁶ = H
und Y = O ist.

Wichtige Zwischenprodukte bei diesen Synthesewegen sind:
(4R,5S)- und (4S,5R)-4-Methyl-5-phenyl-2-oxazolidinon,
   (S)- und (R)-4-Isopropyl-2-oxazolidinon
(4R,5S)- und (4S,5R)-3-Acryloyl-4-methyl-5-phenyl-2-oxazolidinon,
   (4S)- und (4R)-3-Acryloyl-4-isopropyl-2-oxazolidinon,
(4R,5S)-3- und (4S,5R)-3-[1-Oxo-3-halogenpropyl]-4-methyl-5-phenyl-2-oxazolidinon,
   (4R,5S)-3- und (4S,5R)-3-[1-Oxo-3-(alkyl- oder arylsulfonyloxy)propyl]-4-methyl-5-phenyl-2-oxazolidinon,
   (4S)- und (4R)-3-[1-Oxo-3-halogenpropyl]-4-isopropyl-2-oxazolidinon,
   (4S)- und (4R)-3-[1-Oxo-3-(alkyl- oder arylsulfonyloxy)propyl]-4-isopropyl-2-oxazolidinon,
(4R,5S)-3- und (4S,5R)-3-[1-Oxo-2-(alkyl- oder arylthio)methyl]propyl-4-methyl-5-phenyl-2-oxazolidinon,
   (4S) und (4R)-3-[1-Oxo-2-(alkyl- oder arylthio)methyl]propyl-4-isopropyl-2-oxazolidinon,
(4R,5S)-3-[(2S)-1-Oxo-2-(alkyl- oder arylthiomethyl)-3-(alkyl- oder aryl)propyl]-4-methyl-5-phenyl-2-oxazolidinon,
   (4S,5R)-3-[(2R)-1-Oxo-2-(alkyl- oder arylthiomethyl)-3-(alkyl- oder aryl)propyl]-4-methyl-5-phenyl-2-oxazolidinon,
(4S)-3-[(2R)-1-Oxo-2-(alkyl- oder arylthiomethyl)-3-(alkyl- oder aryl)propyl-4-isopropyl-2-oxazolidinon,
   (4R)-3-[(2S)-1-Oxo-2-(alkyl- oder arylthiomethyl)-3-(alkyl- oder aryl)propyl]-4-isopropyl-2-oxazolidinon und
(2S)- und (2R)-2-(Alkyl- oder arylsulfoxymethyl)-3-(alkyl- oder aryl)propionsäure.

Die Acryloyl-Verbindungen der Formeln IVa, IVb, Va oder Vb und die Propionyl-Verbindungen der Formeln VIa, VIb, VIIa oder VIIb können durch vollständige, irreversible Deprotonierung der in guten Ausbeuten leicht zugänglichen Verbindungen der Formeln IIa, IIb, IIIa oder IIIb [D. A. Evans, D. J. Mathre, J. Org. Chem. 50 (1985), 1830; D. A. Evans, J. R. Gage, Org. Synth. 68 (1989), 77; P. G. M. Wuts, L. E. Pruitt, Synthesis 1989, 622] mit Natriumhydrid, BuLi, KOtBu in wasserfreien aprotischen Lösungsmitteln wie Tetrahydrofuran, Dimethoxyethan, tert.-Butylmethylether, Toluol und anschließender Umsetzung mit den entsprechenden Carbonsäurehalogeniden, vorzugsweise den Säurechloriden oder den gemischten Anhydriden (z. B. hergestellt aus Acryl- oder 3-Chlor- oder Brom-, Propionsäure, Triethylamin und Carbonsäurechloriden wie Pivaloylchlorid) bei niedrigen Temperaturen (- 80 °C bis - 20 °C) erhalten werden. Auf die Stabilisierung mit Cu/Cu₂Cl₂ im Falle der Acryloyl-Verbindungen (s. Binger et al., Liebigs Ann. Chem. 1989, 739; Oppolzer et al. Tetrahedron Lett. (1991), 4893) konnte verzichtet werden.

Bevorzugt wird, vor allem für die Synthese der Acryloylverbindungen, die Umsetzung der Oxazolidinone bei Raumtemperatur in Tetrahydrofuran mit Natriumhydrid, anschließende Abkühlung auf - 80 °C bis - 60 °C und Umsetzung mit dem Säurechlorid.

Zur Durchführung der conjugaten Mercaptan-Addition werden die entsprechenden Thioverbindungen gelöst in Ether (bevorzugt THF) mit Alkalimetalhydriden (bevorzugt NaH) oder geeigneten Organometallverbindungen (bevorzugt BuLi) oder mit Fluoriden (z. B. Tetra-n-butylammoniumfluorid) umgesetzt und daraufhin mit den Verbindungen der Formeln IVa, IVb, Va oder Vb zur Reaktion gebracht.

Alternativ dazu läßt sich die Mercaptan-Addition auch phasentransferkatalysiert (z. B. mit Methyltrioctylammoniumchlorid oder Tetra-n-butylammoniumhydrogensulfat) in einer Mischung aus wäßriger NaOH und organischen Lösungsmitteln wie Methylenchlorid, Dichlorethan, Tetrahydrofuran, Dimethoxyethan, tert.-Butylmethylether, Diisopropylether etc. durchführen.

Eine weitere Möglichkeit zur Synthese der Verbindungen VIIIa,b, IXa,b besteht in der Umsetzung von Verbindungen der Formel Vla,b, Vlla,b mit Mercaptanen der Formel R¹SH, worin R¹ die obengenannte Bedeutung hat, in Gegenwart von Basen wie K₂CO₃ in protischen Lösungsmitteln wie Ethanol.

Aufgrund der höheren Gesamtausbeute, der Natur der Nebenprodukte und einer einfacheren, schnelleren Aufarbeitung und Isolierung der Produkte haben sich jedoch "Eintopf-Varianten" als vorteilhaft erwiesen. Diese führen erfindungsgemäß in einem Schritt direkt von Verbindungen der Formeln IIa oder IIb bzw. IIIa oder IIIb zu Verbindungen der Formeln VIIIa oder VIIIb bzw. IXa oder IXb. Dazu setzt man die chiralen Auxiliare der Formeln IIa oder IIb bzw. IIIa oder IIIb mit einer sowohl für die Deprotonierung der Oxazolidinone als auch für das Mercaptan ausreichenden Menge Base (NaH, BuLi etc.) um, gibt nach vollständiger Deprotonierung Acryl- oder Propionsäurehalogenid oder deren jeweiliges gemischtes Anhydrid zu und versetzt die resultierende Mischung nach Ablauf der Acylierung (DC-Kontrolle) mit dem jeweiligen Mercaptan.

Bevorzugt wird weiterhin folgende Vorgehensweise:
Nach Ablauf der NaH- oder BuLi-vermittelten Acylierung der Verbindungen der Formeln IIa, IIb, IIIa oder IIIb mit Acryl- oder Propionsäurehalogenid bzw. mit den jeweiligen gemischten Anhydriden bei - 80 °C bis - 60 °C gibt man Protonendonoren wie z. B. Essigsäure, Citronensäure, Wasser zu, erwärmt auf Raumtemperatur, stellt einen pH-Wert von 9 bis 12 ein (z. B. mit wäßriger NaOH) und gibt das Mercaptan sowie einen Phasentransfer-Katalysator wie z. B. Tetra-n-butylammoniumhydrogensulfat, Methyltrioctylammoniumchlorid hinzu.

Die Alkylierung der Verbindungen der Formeln VIIIa, VIIIb, IXa oder IXb erfolgt vorzugsweise in wasserfreien aprotischen Lösungsmitteln, bevorzugt in THF, durch stereoselektive Enolatbildung (D. A. Evans, Pure Appl. Chem. 53 (1981) 1109; D. A. Evans, Aldrichimica Acta 15 (1982), 23) z. B. bei - 80 °C bis - 60 °C mit lithiierten sekundären Aminen (z. B. LDA) oder Na- oder Li-Hexamethyldisilazan (NaHMDS, LiHMDS) und anschließender Umsetzung mit Alkyl- oder Arylhalogeniden vorzugsweise bei - 50 °C bis - 10 °C.

Die anschließende schonende Abspaltung des chiralen Auxiliars erfolgt mit H₂O₂ in alkalischer Lösung bei - 10 bis 30 °C, bevorzugt mit LiOH/H₂O₂ bei 0 °C bis 25 °C, vorzugsweise in Lösungsmitteln wie Dioxan, THF, Dimethoxyethan oder tBuOMe bzw. wäßrigen Gemischen dieser Lösungsmittel (Evans et al., Tetrahedron Lett. 28 (1987), 6141).

Gegebenenfalls lassen sich die Verbindungen der Formel Xa, Xla, Xb, Xlb durch Umesterung (z. B. mit LiOR, Ti(OR)₄, oder BrMgOR), durch Transaminierung (z. B. mit Me₂AlN(OR)R) oder durch Reduktion z. B. mit LiAlH₄, LiBH₄ in Carbonsäurederivate wie Ester und Amide bzw. in Alkohole überführen. R bedeutet in diesem Zusammenhang Me, Et, CH₂Ph, Isopropyl oder n-Butyl.

Im Falle der LiOH/H₂O₂-induzierten Hydrolyse der Verbindungen der Formeln Xa, Xb, XIa oder XIb führt dies, wie geplant, direkt zu den Sulfoxiden der Formel XII. Die Oxidation zu den Verbindungen der Formeln I ist sowohl direkt als auch nach vorheriger Isolierung der Sulfoxide möglich.

Geeignete Oxidationsmittel sind:
* Metachlorperbenzoesäure (mCPBA), z.B. in CH₂Cl₂ bei 0-25°C;
* Kaliumpermanganat (KMnO₄), z.B. in H₂O/K₂CO₃ bei Raumtemperatur;
* Oxone (2KHSO₅•KHSO₄•K₂SO₄) in Wasser bei Raumtemperatur;
* Monoperoxyphthalsäure-Magnesiumsalz (MMPP) in Wasser bei Raumtemperatur;
* besonders bevorzugt ist H₂O₂, 30 %ig, bei Raumtemperatur in CH₂Cl₂, in Gegenwart von Essigsäure und Polyphophorsäure.

Ein großer verfahrenstechnischer Vorteil, insbesondere für große Ansätze, ergibt sich aus der Zusammenfassung von Alkylierung, Hydrolyse und Oxidation zu einem Eintopf-Verfahren.

Nach direkter Überführung der Verbindungen der Formeln VIIIa, VIIIb, IXa oder IXb in Verbindungen der Formeln XII bzw. I beschränkt sich die Abtrennung der unerwünschten Nebenprodukte und die Entfernung der überschüssigen Reagenzien auf eine einfache extraktive Aufarbeitung der basisch eingestellten Reaktionsmischung (z. B. Verteilung zwischen Wasser und Methylenchlorid). Nach Ansäuern der Wasserphase und Extraktion mit Essigester erhält man die gewünschten Verbindungen der Formeln XII bzw. I in hoher chemischer und optischer Reinheit.

Das erfindungsgemäße Verfahren beinhaltet somit eine Abfolge von maximal 4 Stufen mit Ausbeuten von 60 - 95 % (Gesamtausbeute liegt zwischen 30 und 40 %). Die Aufarbeitung der Zwischen- und Endprodukte erfordert keine Säulenchromatographie, sondern beschränkt sich auf Kristallisation, Extraktion und Filtration. Die verwendeten Ausgangsverbindungen sind preiswert und stellen arbeits- und sicherheitstechnisch kein Problem dar.

### Abkürzungsverzeichnis

- Bu: Butyl
- LDA: Lithiumdiisopropylamid
- Me: Methyl
- OTs: p-Toluolsulfonsäurerest
- OMs: Methylsulfonsäurerest
- Ph: Phenyl
- t: tertiär
- THF: Tetrahydrofuran

Nachfolgende Beispiele sollen die Erfindung näher erläutern:

### Beispiel 1:

### Synthese von Verbindung IVa mit R³ = Methyl, R⁵ = Phenyl

1,8 g (45 mmol) NaH (60 %ig) werden in 50 ml THF vorgelegt und 5 g (28 mmol) Oxazolidinon der Formel IIa mit R³ = Me, R⁵ = Ph in 20 ml THF bei 20 bis 25 °C zugegeben. Es wird eine Stunde bei 20 bis 25 °C gerührt, dann auf - 70 °C gekühlt und 4,7 ml (58 mmol) Acrylsäurechlorid tropfenweise zugegeben. Man hält die Temperatur zwischen - 60 und - 65 °C. Nach 10 Minuten Rühren ist die Umsetzung beendet. Es werden 50 ml gesättigte NaHCO₃-Lösung zugetropft. Die Temperatur steigt dabei auf ca.- 10 °C. Dann entfernt man das Kältebad, gibt Essigester zu, trennt die Phasen, wäscht die organische Phase zweimal mit gesättigter NaCl-Lösung, trocknet mit Na₂SO₄ und destilliert die Lösungsmittel im Vakuum ab. Der Rückstand wird mit tert.-Butylmethylether aufgenommen, der Niederschlag wird abgetrennt. Die verbleibende Lösung (Filtrat) enthält 4,6 g (69 %) des gewünschten Produktes.
¹H-NMR (200 MHz, CDCl₃):
- δ =: 0.95 (d; 3H, CHCH₃);
4.82 (quint; 1H, CHCHCH₃);
5.70 (d; 1H, CHCHPh);
5.92 (dd; 1H, olef.);
6.57 (dd; 1H, olef.);
7.1 - 7.5 (m; 5H, Ph);
7.53 (dd; 1H, olef.).

### Beispiel 2:

### Synthese von Verbindung VIIIa mit R¹ = C(CH₃)₃, R³ = Methyl, R⁵ = Phenyl

1,39 g (6,0 mmol) der Verbindung IVa mit R³ = Me, R⁵ = Ph werden mit 10 ml Toluol, 5 ml Wasser, 1 ml 5 N NaOH, 20 mg Tetra-n-butylammoniumhydrogensulfat und 0,76 ml (6,74 mmol) tert.-Butylmercaptan versetzt und bei Raumtemperatur 2 h gerührt. Nach Zugabe von 100 ml Wasser extrahiert man mit 100 ml Essigester, trocknet die organische Phase mit MgSO₄, rotiert im Vakuum ein und behandelt den Rückstand mit tert.-Butylmethylether. Der entstehende Niederschlag wird abfiltriert und verworfen, die verbleibende Lösung wird eingeengt und man erhält 1,54 g (80 %) der Mercaptoverbindung der Formel Vllla mit R¹ = t-Bu, R³ = Me, R⁵ = Ph.
¹H-NMR (200 MHz, CDCl₃):
- δ =: 0.92 (d; 3H, CHCH₃);
1.35 (s; 9H, C(CH₃)₃);
2.8 - 2.9 (m; 2H, CH₂CH₂);
3.2 - 3.3 (m; 2H, CH₂CH₂);
4.78 (quint; 1H, CHCHCH₃);
5.68 (d; 1H, CHCHPh);
7.25 - 7.50 (m; 5H, Ph).
- Fp:: 65 - 67 °C

### Beispiel 3:

### Synthese von Verbindung Vllla mit R¹ = C(CH₃)₃, R³ = Me, R⁵ = Ph

0,7 g (3,0 mmol) der Verbindung IVa mit R³ = Me, R⁵ = Ph, 0,012 g (0,3 mmol) NaOH in 0,5 ml Wasser, 10,5 mg Tetra-n-butylammoniumhydrogensulfat und 0,39 ml (3,4 mmol) tert.-BuSH werden analog zu Beispiel 2 in 4 - 5 ml Methylenchlorid 2 h bei Raumtemperatur gerührt. Nach Aufarbeitung (Extraktion mit CH₂Cl₂, Trocknung mit MgSO₄) wie in Beispiel 2 erhält man 723 mg (75 %) der Verbindung Vllla mit R³ = Me, R⁵ = Ph.
¹H-NMR (200 MHz, CDCl₃):
- δ =: 0.92 (d; 3H, CHCH₃);
1.35 (s; 9H, C(CH₃)₃);
2.8 - 2.9 (m; 2H, CH₂CH₂);
3.2 - 3.3 (m; 2H, CH₂CH₂);
4.78 (quint; 1H, CHCHCH₃);
5.68 (d; 1H, CHCHPh);
7.25 - 7.50 (m; 5H, Ph).
- Fp:: 65 - 68 °C

### Beispiel 4:

### Synthese von Verbindung VIIIb mit R¹ = C(CH₃)₃, R⁴ = Me, R⁶ = Ph

7,2 g (ca. 180 mmol) NaH (55 - 60 %ig) werden in einem 1-Liter-4-Hals-Kolben mit 200 ml trockenem Tetrahydrofuran vorgelegt. Dazu tropft man bei Raumtemperatur eine Mischung aus 20 g (112,8 mmol) Oxazolidinon der Formel llb mit R⁴ = Me, R⁶ = Ph und 80 ml trockenem THF zu. Die Reaktionsmischung wird eine Stunde bei Raumtemperatur gerührt, dann auf - 60 bis - 65 °C gekühlt und anschließend langsam mit 11,8 ml (146,64 mmol) Acrylsäurechlorid versetzt. Nach 10 minütigem Rühren entfernt man das Kältebad und gibt langsam 120 ml Wasser zu. Mit 5 N NaOH wird nun ein pH-Wert von 11 bis 12 eingestellt.
Bleibt der pH konstant, gibt man 450 bis 500 mg Methyltrioctylammoniumchlorid und 13,16 ml (116 mmol) tert.-Butylmercaptan zu. Nach 30 bis 40 Minuten gibt man ca. 400 ml Wasser und etwa 400 ml Essigester zu, schüttelt aus, trennt die organische Phase ab, extrahiert die wäßrige erneut mit 200 ml Essigester und wäscht die gesammelten organischen Phasen mit gesättigter NaCl-Lösung. Nach Trocknen mit Na₂SO₄ oder MgSO₄ wird die organische Lösung im Vakuum eingedampft und der Rückstand mit ca. 300 ml tert.-Butylmethylether aufgenommen. Nach Filtration wird die etherische Lösung eingeengt. Der Rückstand wird mit n-Heptan/Essigester oder n-Heptan/tert. BuOMe zur Kristallisation gebracht.
Die Ausbeute beträgt nach vollständiger Kristallisation 26,08 - 26,80 g (72 - 74 %).
¹H-NMR (200 MHz, CDCl₃):
- δ =: 0.92 (d; 3H, CHCH₃);
1.35 (s; 9H, C(CH₃)₃);
2.8 - 2.9 (m; 2H, CH₂CH₂);
3.2 - 3.3 (m; 2H, CH₂CH₂);
4.78 (quint; 1H, CHCHCH₃);
5.68 (d; 1H, CHCHPh);
7.25 - 7.50 (m; 5H, Ph).
- Fp:: 68 - 70 °C

### Beispiel 5:

### Synthese von Verbindung Vllla mit R¹ = C(CH₃)₃, R³ = Me, R⁵ = Ph

1,8 g (ca. 45 mmol) NaH (60 %ig) werden mit Petrolether gewaschen und anschließend mit 50 ml THF versetzt. Dann löst man 5 g (28,2 mmol) Oxazolidinon der Formel IIa mit R³ = Me, R⁵ = Ph, in 20 ml THF und tropft diese Lösung in die NaH/THF-Mischung. Nach einstündigem Rühren bei Raumtemperatur kühlt man die Lösung auf - 20 °C, tropft langsam 2,35 ml (29,03 mmol) Acrylsäurechlorid zu und rührt weitere 5 Minuten.
Anschließend gibt man 18 ml Wasser zu, erwärmt die Mischung auf Raumtemperatur, stellt einen pH-Wert von 10 ein, gibt 75 mg Tetra-n-butylammoniumhydrogensulfat zu und gibt 2,87 ml (25,45 mmol) tert.-Butylmercaptan zu. Nach 2 Stunden Rühren bei Raumtemperatur gibt man 300 ml Wasser und 250 ml Essigester zu. Nach Extraktion und Trocknung mit Na₂SO₄ wird die organische Lösung im Vakuum eingeengt. Der Rückstand wird mit tert.-Butylmethylether verrührt. Der sich bildende feine Niederschlag wird abfiltriert, die etherische Lösung wird zur Trockne eingedampft. Nach Filtration über 10 - 15 g Kieselgel (in CH₂Cl₂) und nach Umkristallisieren aus Petrolether erhält man 5,8 - 6,0 g (64 - 66 %) reines Michael-Addukt der Formel Vllla mit R' = C(CH₃)₃, R³ = Me, R⁵ = Ph.
¹H-NMR (200 MHz, CDCl₃):
- δ =: 0.92 (d; 3H, CHCH₃);
1.35 (s; 9H, C(CH₃)₃);
2.8 - 2.9 (m; 2H, CH₂CH₂);
3.2 - 3.3 (m; 2H, CH₂CH₂);
4.78 (quint; 1H, CHCHCH₃);
5.68 (d; 1H, CHCHPh);
7.25 - 7.50 (m; 5H, Ph).
- Fp:: 73 - 75 °C

### Beispiel 6:

### Synthese von Verbindung Vlllb mit R¹ = C(CH₃)₃, R⁴ = Me, R⁵ = Ph

0,676 g (16,92 mmol) NaH (60 %) werden mit 25 ml THF (trocken) vorgelegt. Dazu tropft man bei Raumtemperatur eine Lösung aus 2,5 g (14,1 mmol) Oxazolidinon der Formel IIb mit R⁴ = Me, R⁶ = Ph, in 10 ml THF (trocken). Nach 1 h Rühren bei Raumtemperatur kühlt man auf - 60 °C und tropft langsam 1,19 ml (14,8 mmol) Acrylsäurechlorid zu. Nach ca. 10 Minuten ist die Reaktion beendet. Nun tropft man eine vorher hergestellte Mischung aus 2,0 ml (18,33 mmol) t-Butylmercaptan und 19,2 ml (21,15 mmol) einer 1,1 molaren Tetra-n-butylammoniumfluorid-Lösung in THF zu. Nach 2 h bei - 60 °C gibt man 150 ml Wasser und 150 ml Essigester hinzu, schüttelt aus, trocknet die organische Phase mit MgSO₄ und entfernt das Lösungsmittel im Vakuum. Der Rückstand wird in tert.-Butylmethylether gelöst, der ausgefallene feine Niederschlag wird über eine Klärschicht abgesaugt und verworfen und die etherische Phase wird im Vakuum eingeengt. Der Rückstand wird langsam fest. Nach Umkristallisieren oder Filtration über wenig Kieselgel (in CH₂Cl₂) erhält man 3,4 g (75 %) des gewünschten Michael-Adduktes der Formel VIIIb mit
R¹ = C(CH₃)₃.
¹H-NMR (200 MHz, CDCl₃):
- δ =: 0.92 (d; 3H, CHCH₃);
1.35 (s; 9H, C(CH₃)₃);
2.8 - 2.9 (m; 2H, CH₂CH₂);
3.2 - 3.3 (m; 2H, CH₂CH₂);
4.78 (quint; 1H, CHCHCH₃);
5.68 (d; 1H, CHCHPh);
7.25 - 7.50 (m; 5H, Ph).
- Fp:: 69 - 73 °C

### Beispiel 7:

### Synthese von Verbindung Vlllb mit R¹ = C(CH₃)₃, R⁴ = Me, R⁶ =Ph

2,82 g (70,5 mmol) NaH (60 %ig) werden mit Petrolether gewaschen. Dann gibt man 50 ml getrocknetes Tetrahydrofuran zu und tropft dann unter Rühren eine Lösung von 5 g (28,2 mmol) Oxazolidinon der Formel IIb mit R⁴ = Me, R⁶ = Ph, in 20 ml THF bei Raumtemperatur zu. Nach einstündigem Rühren bei Raumtemperatur kühlt man auf - 60 °C ab und gibt langsam 2,35 ml (29 mmol) Acrylsäurechlorid zu und rührt weitere 10 Minuten. Nun gibt man bei - 60 °C 3,49 ml (31 mmol) tert.-Butylmercaptan hinzu. Nach ca. 1,0 - 1,5 Stunden wird die Reaktionsmischung langsam in 100 ml gesättigte Ammoniumchlorid-Lösung gegeben. Nach Aufwärmen auf Raumtemperatur werden 200 ml Wasser und 300 ml Essigester zugegeben. Man schüttelt aus, trocknet die abgetrennte organische Phase mit MgSO₄ und engt die Lösung im Vakuum ein. Der verbleibende Rückstand wird mit tert.-Butylmethylether versetzt, die Trübung durch Filtration entfernt, die klare etherische Lösung wird anschließend eingedampft. Der Rückstand kristallisiert in der Kälte. Nach Filtration über wenig Kieselgel und Umkristallisieren aus n-Heptan erhält man 5,89 g (65 %) der gewünschten Verbindung VIIIb mit R¹ = C(CH₃)₃, R⁴ = Me, R⁶ = Ph.
¹H-NMR (200 MHz, CDCl₃):
- δ =: 0.92 (d; 3H, CHCH₃);
1.35 (s; 9H, C(CH₃)₃);
2.8 - 2.9 (m; 2H, CH₂CH₂);
3.2 - 3.3 (m; 2H, CH₂CH₂);
4.78 (quint; 1H, CHCHCH₃);
5.68 (d; 1H, CHCHPh);
7.25 - 7.50 (m; 5H, Ph).
- Fp:: 73 - 75 °C

### Beispiel 8:

### Synthese von Verbindung (S)-XII mit R¹ = C(CH₃)₃ und R² = 1-Naphthyl

0,59 ml (4,2 mmol) Diisopropylamin werden in 7 ml THF vorgelegt. Bei - 60 °C werden 2,5 ml (4,04 mmol) BuLi (1,6 M in Hexan) zugetropft. Man rührt 10 Minuten bei - 60 °C und tropft dann eine Lösung aus 1 g (3,11 mmol) der Verbindung der Formel Vllla mit R¹ = tBu, R³ = Me, R⁵ = Ph, in 3 ml THF zu. Man rührt weitere 20 Minuten und gibt dann tropfenweise eine Lösung aus 1,37 g (6,22 mmol) 1-Brommethylnaphthalin in 3 ml THF hinzu. Man rührt 5 Minuten bei - 60 °C. Anschließend läßt man durch Entfernen des Kühlbades innerhalb von 15 Minuten die Temperatur auf - 10 °C ansteigen. Nach 40 - 60 Minuten ist kein Edukt mehr vorhanden und man gibt
0,40 g (9,5 mmol) LiOH•H₂O und 1,0 ml (12 mmol) H₂O₂ (35 %ig) in 1 ml H₂O hinzu. Man rührt ca. 30 Minuten bei Raumtemperatur, dampft das THF im Vakuum ein, extrahiert die verbleibende Reaktionsmischung mit CH₂Cl₂. Anschließend säuert man die wäßrige Phase auf pH = 1 an und extrahiert gründlich mit Essigester. Die organische Phase wird mit Na₂SO₄ getrocknet und eingeengt. Man erhält 594 mg (60 %) des gewünschten Produktes.
¹H-NMR (200 MHz, d₆-DMSO):
- δ =: 1.10, 1.15 (s; 9H, SC(CH₃)₃);
2.70 - 3.70 (m; 5H, CH u. CH₂);
7.1 - 8.2 (m; 7H, aromat.);
12.6 (br; 1H, COOH).

### Beispiel 9:

### Überführung des Alkylierungsproduktes der Formel Xb mit R⁴ = Me, R⁶ = Ph, R¹ = tBu in die (2R)-Sulfoxylcarbonsäure der Formel XII mit R¹ = C(CH₃)₃ und R² = 1-Naphthyl

200 mg (0,43 mmol) der Verbindung Xb mit R⁴ = Me, R⁶ = Ph, R¹ = tBu, R² = 1-Naphthyl werden in 2 ml THF gelöst und mit 0,11 ml (1,3 mmol) H₂O₂ (35 %ig) und 55 mg (1,3 mmol) LiOH•H₂O in 1 ml Wasser versetzt und bei Raumtemperatur gerührt. Nach 30 - 40 Minuten ist die Reaktion beendet. Nach Abtrennen der THF-Phase extrahiert man die Reaktionsmischung mit CH₂Cl₂ (Rückgewinnung des chiralen Auxiliars), säuert die wäßrige Phase dann an (pH = 1) und extrahiert die gewünschte Carbonsäure mit Essigester. Nach Trocknen (Na₂SO₄) und Einengen im Vakuum erhält man 126 mg (92 %) der gewünschten (2R)-Sulfonylcarbonsäure XII.
MS (70 eV): m/z (%) =
319.2 (100, M^{⊕} +1);
262.2 ( 25, M-C₄H₈).

### Beispiel 10:

### Oxidation des (2S)-Sulfoxids der Formel XII mit R¹ = C(CH₃)₃, R² = Naphthyl zum (2S)-Sulfon der Formel I (R¹ = C(CH₃)₃, R² = Naphthyl)

500 mg (1,57 mmol) (S)-Sulfoxid XII (mit R¹ = C(CH₃)₃ und R² = Naphthyl) werden in 1 ml CH₂Cl₂ gelöst. Man gibt nacheinander 0,3 ml Eisessig, 0,52 ml H₂O₂ sowie 0,16 g Polyphosphorsäure hinzu.
Nach 2 h Rühren ist die Reaktion beendet. Man versetzt das Reaktionsgemisch mit Wasser und trennt die Phasen. Die organische Phase wird zweimal mit Wasser gewaschen, getrocknet (Na₂SO₄) und einrotiert. Man erhält 444 mg (84,7 %) der gewünschten (S)-konfigurierten Verbindung I.
¹H-NMR (270 MHz, d₆-DMSO)
- δ =: 1.25 (s; 9H, O₂SC(CH₃)₃);
3.1 - 3.65 (m; 5H, CH₂ u. CH);
7.35 - 8.2 (m; 7H, aromat.);
12.55 (br.; 1H, COOH).
MS (70 eV) : m/z (%) =
334 (100, M^{⊕}).

### Beispiel 11

### Oxidation des (2S)-Sulfoxids der Formel XII mit R¹ = C(CH₃)₃, zum (2S)-Sulfon der Formel I(R¹ = C(CH₃)₃), R² = Phenyl)

Zu einer wäßrigen Lösung (10 ml) von 1,34 g (5 mmol) des genannten (S)-Sulfoxids Formel XII und 300 - 500 mg K₂CO₃ werden 790 mg (5 mmol) KMnO₄ in 20 ml Wasser bei Raumtemperatur zugetropft. Nach beendeter Reaktion (DC-Kontrolle) wurde ausgefallener Braunstein abfiltriert, die wäßrige Lösung angesäuert und das gewünschte Sulfon mit Essigester extrahiert. Nach Trocknen (Na₂SO₄) und Einengen der organischen Lösung im Vakuum erhält man 1,25-1,35 g (88-95 %) der gewünschten (S)-konfigurierten Verbindung I mit R¹ = t-Bu, und R² = Phenyl.
¹HNMR (200 MHz, CDCl₃)
- δ =: 1.3 (s; 9H, O₂S C(CH₃)₃);
2.9 - 3.6 (m; 5H, CH₂ n. CH);
7.15 - 7.35 (m; 5H, Ph).
MS (70 eV) : m/z =
284 (M^{⊕})

### Beispiel 12:

### Oxidation des (2R)-Sulfoxids der Formel XII mit R¹ = C(CH₃)₃, R² = Phenyl, zum (2R)-Sulfon der Formel I (R¹ = tBu, R² = Phenyl)

670 mg (2,5 mmol) des (R)-Sulfoxids der Formel XII werden in 5 - 10 ml t-Butylmethylether und 2 - 5 ml Wasser vorgelegt und bei 0 °C mit 2,5 g Oxone in 10 ml Wasser tropfenweise versetzt. Anschließend rührt man bei Raumtemperatur. Nach Ablauf der Reaktion (DC-Kontrolle) und Ansäuern der Reaktionsmischung werden die Phasen getrennt. Die wäßrige Phase wird zweimal mit Essigester extrahiert und die vereinigten organischen Phasen werden mit MgSO₄ getrocknet. Man erhält 682 mg (96 %) des gewünschten (R)-konfigurierten Sulfons der Formel I mit R¹ = C(CH₃)₃ und R² = Phenyl.
¹H-NMR (200 MHz, CDCl₃)
- δ =: 1.3 (s; 9H, O₂S C(CH₃)₃);
2.9 - 3.6 (m; 5H, CH₂ n. CH);
7.15 - 7.35 (m; 5H, Ph).
MS (70 eV) : m/z =
284 (M^{⊕})

### Beispiel 13:

### Synthese von (4S,5R)-3-(1-Oxo-3-chlorpropyl)-4-methyl-5-phenyl-2-oxazolidinon (Verbindung der Formel Vlb mit R⁴ = Methyl, R⁶ = Phenyl, X = Cl)

0,52 g (12 mmol) NaH (60 %) werden in 2 ml THF vorgelegt. Unter Rühren tropft man dazu eine Lösung aus 1,32 g (7,45 mmol) (4S,5R)-4-Methyl-5-phenyl-2-oxazolidinon in 13 ml THF. Nach 30 - 40 minütigen Rühren bei Raumtemperatur tropft man 1,23 g (9,7 mmol) 3-Chlorpropionsäurechlorid bei - 60 °C langsam hinzu und rührt dann ca. 30 min. bei - 60 °C. Anschließend gibt man bei - 60 bis - 40 °C 10 bis 15 ml Citronensäure/Wasser zu, läßt die Reaktionstemperatur langsam auf 25 °C ansteigen, schüttelt mit Essigester aus, trocknet die organische Phase mit MgSO₄ und engt im Vakuum ein. Nach Kristallisation erhält man 1,3 - 1,4 g (65 - 70 %) des gewünschten Produkts.
¹H-NMR (200 MHz, CDCl₃):
- δ =: 0.92 (d; 1H, CHCH₃);
3.4 - 3.5 (m; 2H, CH₂CH₂);
3.8 - 3.9 (m; 2H, CH₂CH₂);
4.80 (quint; 1H, CHCHCH₃);
5.70 (d; 1H, CHCHPh);
7.2 - 7.5 (m; 5H, Ph).

### Beispiel 14:

### Fällung von Verbindungen der Formel I als Cyclohexylammoniumsalz

21 g I werden in 200 ml Aceton gelöst und mit 6 g Cyclohexylamin versetzt. Nach Verdünnen mit 100 ml Aceton wird abgesaugt. Man erhält 20,4 g I-Cyclohexylammoniumsalz.

### Beispiel 15:

### Freisetzung von Verbindungen der Formel I aus dem Cyclohexylammoniumsalz

15 g des Salzes werden mit 100 ml Essigester und 100 ml 2N HCI versetzt und ausgeschüttelt. Die organische Phase wird mit 100 ml ges. NaCl-Lösung gewaschen, mit Na₂SO₄ getrocknet und eingeengt. Man erhält 12 g der freien Säure I.

### Beispiel 16:

### Unterscheidung von (S)-I und (R)-I

(S)-I und/oder (R)-I werden in Methylenchlorid gelöst auf 0 °C gekühlt und mit einem Überschuß Triethylamin, (S)-Phenylalaninol und Propanphosphonsäureanhydrid versetzt und anschließend etwa 1 h bei Raumtemperatur gerührt. Die diastereomeren Amide lassen sich sowohl per HPLC als auch per DC unterscheiden:
Z. B. bei I mit R¹ = tBu, R² = Naphthyl:
(S)-I-(S)-Phenylalaninolamid R_{f} (tBuOMe) 0,60
(R)-I-(S)-Phenylalaninolamid R_{f} (tBuOMe) 0,32

### Beispiel 17:

### Synthese von Verbindung Vllla mit R¹ = C(CH₃)₃, R³ = Me, R⁵ = Ph

0,515 g (11,8 mmol) NaH (ca. 60 %) werden mit 2 ml abs. THF versetzt. Dann gibt man 1,32 g (7,45 mmol) Oxazolidinon IIa mit R³ = Me, R⁵ = Ph zu und rührt 0,5 - 1,0 h bei Raumtemperatur. Nach Abkühlen auf - 60 °C werden 0,72 ml (7,45 mmol) 3-Chlorpropionsäurechlorid langsam zugegeben.
Die Reaktionsmischung wird 30 - 45 min. bei - 60 °C gerührt und anschließend mit einer Mischung aus 20 mg Tetrabutylammoniumhydrogensulfat, 15 ml Wasser und 0,86 ml (7,66 mmol) tert.-Butylmercaptan versetzt. Man läßt das Reaktionsgemisch langsam auf Raumtemperatur aufwärmen, stellt einen pH von 9 - 11 ein und rührt 1 bis 2 Stunden bei Raumtemperatur.
Anschließend wird, nach Zugabe von 50 bis 100 ml Wasser, mit Essigester extrahiert, die organische Phase getrocknet und eingeengt; Rohprodukt: 2,3 g.

Nach Filtration über ca. 6 g Kieselgel und Kristallisation aus Cyclohexan erhält man 1,7 g (73 %) der gewünschten Verbindung VIII mit R¹ = C(CH₃)₃.

Spektroskopische Daten: ¹H-NMR (200 MHz, CDCl₃):
- δ =: 0.92 (d; 3H, CHCH₃);
1.35 (s; 9H, C(CH₃)₃);
2.8 - 2.9 (m; 2H, CH₂CH₂);
3.2 - 3.3 (m; 2H, CH₂CH₂);
4.78 (quint; 1H, CHCHCH₃);
5.68 (d; 1H, CHCHPh);
7.25 - 7.50 (m; 5H, Ph).

### Beispiel 18:

### Synthese von Verbindung Vllla mit R¹ = C(CH₃)₃, R³ = Me, R⁵ = Ph

2,6 g (59 mmol) NaH (60 %ige Dispersion), 6,6 g (37,25 mmol) Oxazolidinon der Formel lla mit R³ = Me, R⁵ = Ph werden eine Stunde bei Raumtemperatur in 10 ml abs. THF gerührt. Man kühlt anschließend auf - 60 °C ab, tropft 3,58 ml (37,25 mmol) Chlorpropionsäurechlorid zu und rührt 30 - 45 min. bei - 60 °C. Nach Zugabe von 1,3 g (6,8 mmol) trockener Citronensäure, rührt man 5 - 15 min. bei - 60 °C, gibt dann 50 ml Wasser langsam zu, erhöht die Temperatur auf - 10 ° bis O °C, gibt 100 mg nBU₄NHSO₄ zu und stellt mit 5N NaOH einen pH von 9 - 10 ein. Nach Zugabe von 4,31 ml (38,6 mmol) tert.-Butylmercaptan rührt man 2 h bei Raumtemperatur. Zur Aufarbeitung der Reaktion gibt man 200 ml Essigester und 200 ml halbgesättigte Citronensäurelösung zu, schüttelt aus, wäscht die organische Phase mit Wasser, trocknet mit MgSO₄ und engt im Vakuum ein;
Rohausbeute 12 g. Nach Filtration über ca. 25 g Kieselgel (CH₂Cl₂) und Ausrühren mit Hexan oder Pentan erhält man 7,8 g (65 %) des gewünschten Produktes.
Fp: 73 - 75 °C

## Patentansprüche

1. Verfahren zur stereoselektiven Herstellung einer Verbindung der Formel I 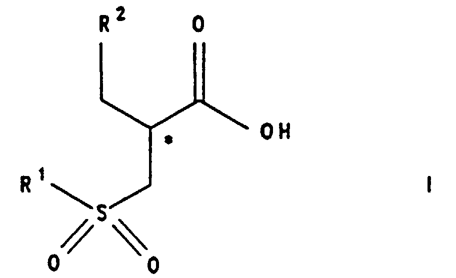 worin
R¹ C₁-C₆-Alkyl, C₃-C₆-Cycloalkyl, (C₆-C₁₂)-Aryl-(C₁-C₄)-alkyl oder C₆-C₁₂-Aryl bzw. -Heteroaryl bzw. -Heterocycloalkyl, die mit 1, 2 oder 3 gleichen oder verschiedenen Hydroxy-, Methoxy- oder Trialkylsilyloxygruppen substituiert sein können; und
R² C₆-C₁₂-Aryl, das mit 1, 2 oder 3 gleichen oder verschiedenen Methoxy-, Halogen-, Cyano-, Methyl-, Trifluormethyl-, Isopropyl- oder Nitrogruppen substituiert sein kann;
C₃-C₉-Heteroaryl, das mit 1, 2 oder 3 gleichen oder verschiedenen Methoxy-, Halogen-, Cyano-, Methyl-, Trifluormethyl, Isopropyl- oder Nitrogruppen substituiert sein kann; oder
C₁-C₁₀-Alkyl, Alkenyl oder Alkinyl, die durch 1, 2 oder 3 gleiche oder verschiedene Methoxy-, Halogen-, Cyano-, Methyl-, Trifluormethyl, Isopropyl- oder Nitrogruppen substituiert sein können, bedeuten
und die Verbindungen der Formel I in der R- oder S-Form vorliegen können, dadurch gekennzeichnet, daß man eine Verbindung der Formel lla oder llb bzw. IIIa oder IIIb (chirales Auxiliar), worin Y = O oder S ist, 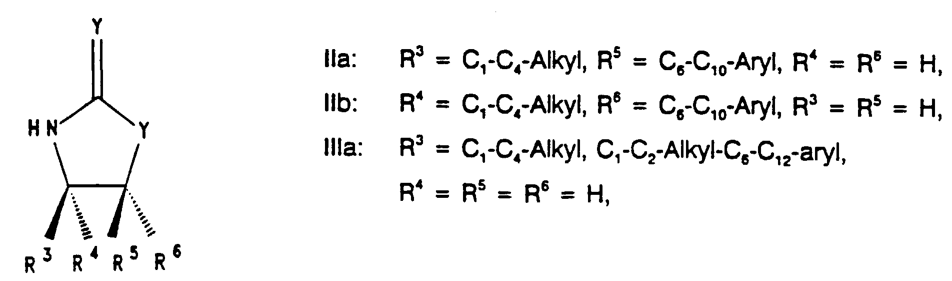 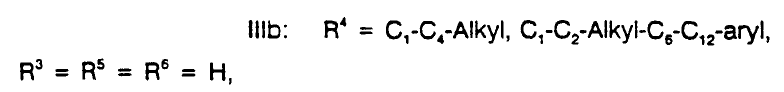
a₁) mit Acrylsäure oder mit Derivaten der Acrylsäure in eine Verbindung der Formel IVa oder IVb bzw. Va oder Vb, worin Y = O oder S ist, 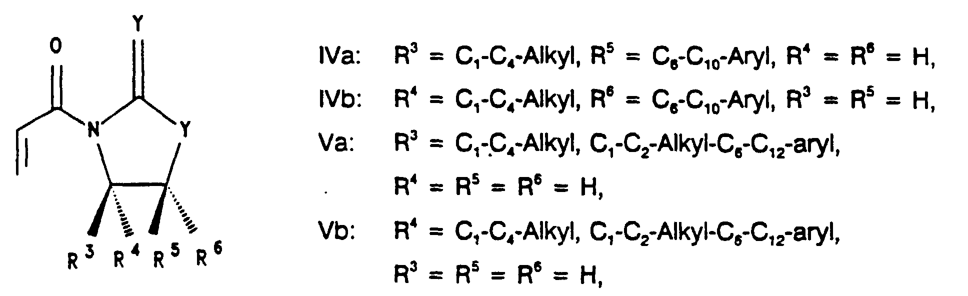 überführt, oder
a₂) mit Propionsäure oder Propionsäurederivaten, die ihrerseits in C₃-Position durch einen Rest X = Cl, Br, OTs, OMs substituiert sind, in eine Verbindung der Formel Vla oder Vlb bzw. Vlla oder Vllb, worin X und Y die obengenannte Bedeutung haben, 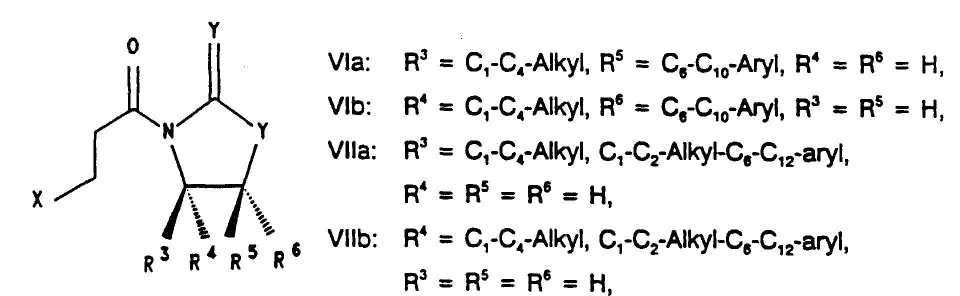 überführt,
b) die nach a₁) oder a₂) erhaltene Verbindung der Formel IVa, IVb, Va, Vb, VIa, VIb, VIIa oder VIIb direkt oder nach Isolierung in nichtwäßrigem Milieu oder in Gegenwart von Phasentransfer-Katalysatoren in wäßrigen Zweiphasen-Systemen mit einem Mercaptan der Formel R¹SH, worin R¹ wie oben definiert ist, in die entsprechende Verbindung der Formel Vllla oder VIIIb bzw. IXa oder IXb, worin R¹ und Y die obengenannte Bedeutung haben, 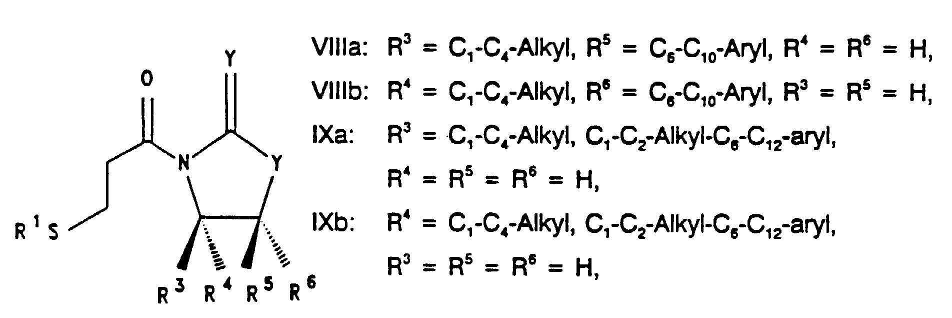 umsetzt, oder
a₃) eine Verbindung der Formel IIa oder IIb bzw. IIIa oder III in Gegenwart einer für die Deprotonierung des Oxazolidinons sowie des Mercaptans R¹SH, worin R¹ obengenannte Bedeutung hat, ausreichenden Menge Base durch Umsetzung mit Acryl- oder Propionsäurehalogeniden oder deren jeweiligen gemischten Anhydriden und weiterer Umsetzung des Reaktionsproduktes mit dem Mercaptan in einem Schritt in eine Verbindung der Formel Vllla oder VIIIb bzw. IXa oder IXb überführt,
c) die nach b) oder a₃) erhaltene Verbindung anschließend durch stereoselektive Alkylierung in die entsprechende Verbindung der Formel Xa, Xb, Xla oder Xlb überführt, 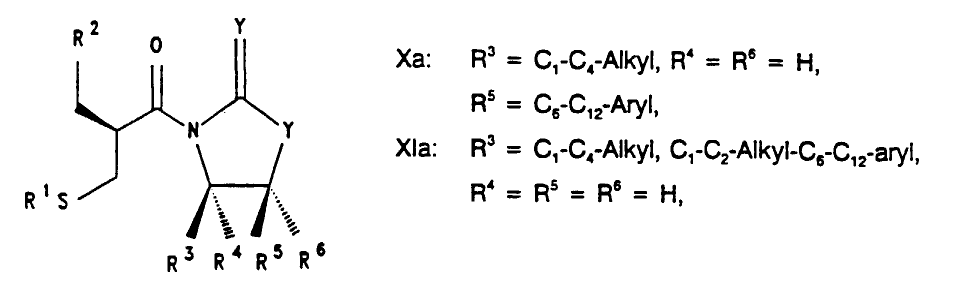 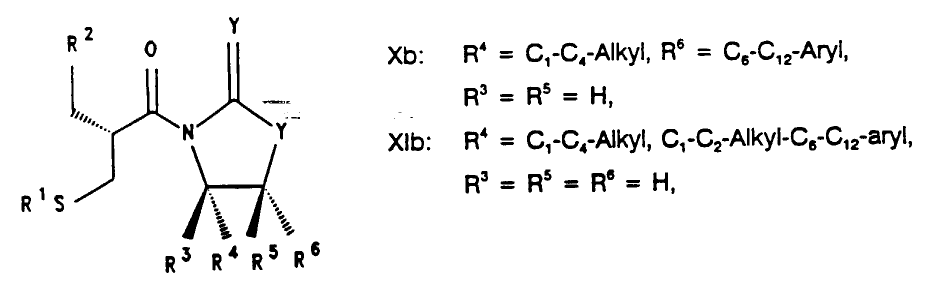 worin Y, R¹ und R² die obengenannte Bedeutung haben,
d) die derart gewonnene Verbindung dann durch Umsetzung mit H₂O₂, in alkalischer Lösung, bevorzugt mit LiOH/H₂O₂, in Verbindungen der Formel XII, die in der R- oder S-Form vorliegen können, 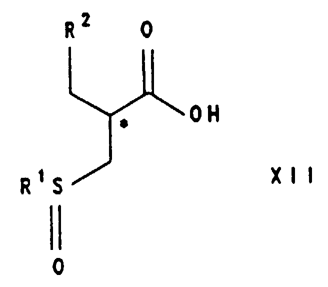 überführt und
e) die so erhaltene Verbindung dann durch weitere Oxidation in die entsprechende Verbindung der Formel I überführt.

2. Verfahren gemäß Anspruch 1, dadurch gekennzeichnet, daß
R¹ C₁-C₄-Alkyl, C₅-C₆-Cycloalkyl, (C₆-C₁₂-Aryl-(C₁-C₃)-alkyl oder C₆-C₁₂-Aryl bzw. Heteroaryl, die mit einer Hydroxy-, Methoxy- oder Trialkylsilyloxygruppe substituiert sein können;
R² C₆-C₁₂-Aryl, das mit einer Methoxy-, Halogen-, Methyl-, Trifluormethyl-, oder Isopropylgruppe substituiert sein kann;
C₃-C₆-Heteroaryl, das mit einer Methoxy-, Halogen-, Methyl-, Trifluormethyl, oder Isopropylgruppe substituiert sein kann oder
C₁-C₄-Alkyl, -Alkenyl oder -Alkinyl, bedeuten.

3. Verfahren gemäß den Ansprüchen 1 oder 2, dadurch gekennzeichnet, daß
R¹ C₁-C₄-Alkyl,
(C₆-C₁₂)-Aryl-(C₁-C₃)-alkyl oder C₆-C₁₂-Aryl und
R² = C₆-C₁₂-Aryl, C₃-C₆-Heteroaryl oder C₁-C₄-Alkyl, -Alkenyl oder -Alkinyl bedeuten.

4. Verfahren gemäß Anspruch einem oder mehreren der Anspruch 1 - 3, dadurch gekennzeichnet, daß eine Verbindung der Formel lla oder llb bzw. IIIa oder IIIb, worin
IIa: R³ = Me, R⁵ = Ph, R⁴ = R⁶ = H
IIb: R⁴ = Me, R⁶ = Ph, R³ = R⁵ = H
IIIa: R³ = CH(CH₃)₂, R⁴ = R⁵ = R⁶ = H
IIIb: R⁴ = CH(CH₃)₂, R³ = R⁵ = R⁶ = H
und Y = O ist eingesetzt werden.

5. Verfahren gemäß einem oder mehreren der Ansprüche 1 - 4, dadurch gekennzeichnet, daß die Verbindung der Formel IIa oder IIb bzw. IIIa oder IIIb gemäß a₃) in einem Schritt direkt in eine Verbindung der Formel VIIIa oder VIIIb bzw. IXa oder IXb überführt wird.

6. Verfahren gemäß einem oder mehreren der Ansprüche 1 - 5, dadurch gekennzeichnet, daß die Alkylierung der Verbindung der Formel Vllla, Vlllb, IXa oder IXb, die anschließende Abspaltung des chiralen Auxiliars sowie die Oxidation zu den Verbindungen der Formel XII bzw. I ohne Isolierung der Zwischenstufen durchgeführt wird.

## Claims

1. A process for the stereoselective preparation of a compound of the formula I 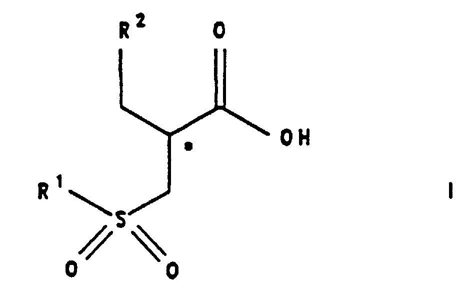 in which
R¹ is C₁-C₆-alkyl, C₃-C₆-cycloalkyl, (C₆-C₁₂) - aryl-(C₁-C₄)-alkyl or C₆-C₁₂-aryl or -heteroaryl or - heterocycloalkyl which may be substituted by 1, 2 or 3 identical or different hydroxyl, methoxy or trialkylsilyloxy groups; and
R² is C₆-C₁₂-aryl which may be substituted by 1, 2 or 3 identical or different methoxy, halogen, cyano, methyl, trifluoromethyl, isopropyl or nitro groups;
is C₃-C₉-heteroaryl which may be substituted by 1, 2 or 3 identical or different methoxy, halogen, cyano, methyl, trifluoromethyl, isopropyl or nitro groups; or
is C₁-C₁₀-alkyl, -alkenyl or -alkynyl which may be substituted by 1, 2 or 3 identical or different methoxy, halogen, cyano, methyl, trifluoromethyl, isopropyl or nitro groups
and the compounds of the formula I may be present in the R or S form, which comprises converting a compound of the formula IIa or IIb or IIIa or IIIb, respectively, (chiral auxiliary), in which Y is = O or S, 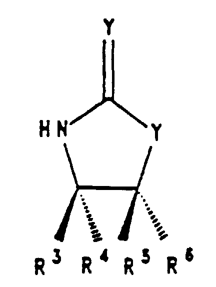
IIa: R³ = C₁-C₄-alkyl, R⁵ = C₆-C₁₀-aryl, R⁴ = R⁶ = H,
IIb: R⁴ = C₁-C₄-alkyl, R⁶ = C₆-C₁₀-aryl, R³ = R⁵ = H,
IIIa: R³ = C₁-C₄-alkyl, C₁-C₂-alkyl-C₆-C₁₂-aryl, R⁴ = R⁵ = R⁶ = H,
IIIb: R⁴ = C₁-C₄-alkyl, C₁-C₂-alkyl-C₆-C₁₂-aryl, R³ = R⁵ = R⁶ = H,
a₁) with acrylic acid or with derivatives of acrylic acid, into a compound of the formula IVa or IVb or Va or Vb, respectively, in which Y is = O or S, 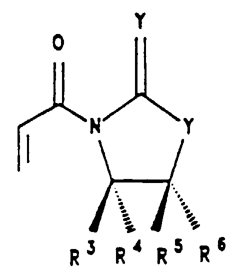
IVa: R³ = C₁-C₄-alkyl, R⁵ = C₆-C₁₀-aryl, R⁴ = R⁶ = H,
IVb: R⁴ = C₁-C₄-alkyl, R⁶ = C₆-C₁₀-aryl, R³ = R⁵ = H,
Va: R³ = C₁-C₄-alkyl, C₁-C₂-alkyl-C₆-C₁₂-aryl, R⁴ = R⁵ = R⁶ = H,
Vb: R⁴ = C₁-C₄-alkyl, C₁-C₂-alkyl-C₆-C₁₂-aryl, R³ = R⁵ = R⁶ = H,
or
a₂) with propionic acid or propionic acid derivatives which are in turn substituted in position C₃ by a radical X = Cl, Br, OTs, OMs, into a compound of the formula VIa or VIb or VIIa or VIIb, respectively, in which X and Y are as defined above, 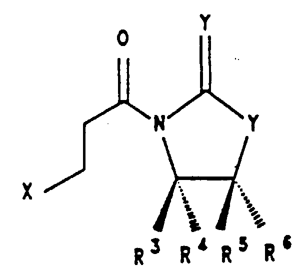
VIa: R³ = C₁-C₄-alkyl, R⁵ = C₆-C₁₀-aryl, R⁴ = R⁶ = H,
VIb: R⁴ = C₁-C₄-alkyl, R⁶ = C₆-C₁₀-aryl, R³ = R⁵ = H,
VIIa: R³ = C₁-C₄-alkyl, C₁-C₂-alkyl-C₆-C₁₂-aryl, R⁴ = R⁵ = R⁶ = H,
VIIb: R⁴ = C₁-C₄-alkyl, C₁-C₂-alkyl-C₆-C₁₂-aryl, R³ = R⁵ = R⁶ = H,
b) reacting the compound of the formula IVa, IVb, Va, Vb, VIa, VIb, VIIa or VIIb obtained according to a₁) or a₂), directly or after isolation in a nonaqueous medium or in the presence of phase-transfer catalysts in aqueous two-phase systems, with a mercaptan of the formula R¹SH, in which R¹ is as defined above, into the corresponding compound of the formula VIIIa or VIIIb or IXa or IXb, respectively, in which R¹ and Y are as defined above, 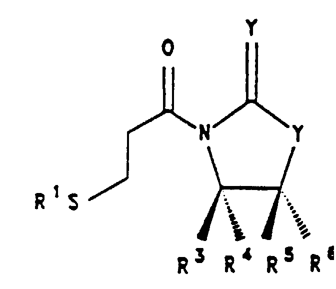
VIIIa: R³ = C₁-C₄-alkyl, R⁵ = C₆-C₁₀-aryl, R⁴ = R⁶ = H,
VIIIb: R⁴ = C₁-C₄-alkyl, R⁶ = C₆-C₁₀-aryl, R³ = R⁵ = H,
IXa: R³ = C₁-C₄-alkyl, C₁-C₂-alkyl-C₆-C₁₂-aryl, R⁴ = R⁵ = R⁶ = H,
IXb: R⁴ = C₁-C₄-alkyl, C₁-C₂-alkyl-C₆-C₁₂-aryl, R³ = R⁵ = R⁶ = H,
or
a₃) converting a compound of the formula IIa or IIb or IIIa or IIIb, respectively, in the presence of a quantity of base which is sufficient for the deprotonation of the oxazolidinone and of the mercaptan R¹SH, in which R¹ is as defined above, into a compound of the formula VIIIa or VIIIb or IXa or IXb, respectively, by reaction with acryloyl or propionyl halides or their respective mixed anhydrides and further reaction of the reaction product with the mercaptan in one step,
c) then converting the compound obtained according to b) or a₃) into the corresponding compound of the formula Xa, Xb, XIa or XIb by stereoselective alkylation, 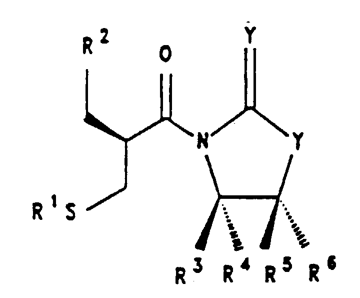
Xa: R³ = C₁-C₄-alkyl, R⁴ = R⁶ = H, R⁵ = C₆-C₁₂-aryl,
XIa: R³ = C₁-C₄-alkyl, C₁-C₂-alkyl-C₆-C₁₂-aryl, R⁴ = R⁵ = R⁶ = H, 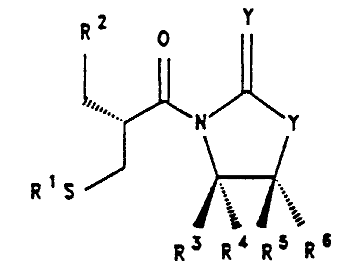
Xb: R⁴ = C₁-C₄-alkyl, R⁶ = C₆-C₁₂-aryl, R³ = R⁵ = H,
XIb: R⁴ = C₁-C₄-alkyl, C₁-C₂-alkyl-C₆-C₁₂-aryl, R³ = R⁵ = R⁶ = H,
in which Y, R¹ and R² are as defined above,
d) then converting the compound obtained in this way by reaction with H₂O₂ in alkaline solution, preferably with LiOH/H₂O₂, into compounds of the formula XII which may be present in the R or S form, 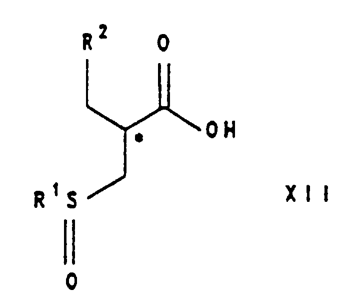 and
e) then converting the compound obtained in this way, by further oxidation, into the corresponding compound of the formula I.

2. The process as claimed in claim 1, wherein
R¹ is C₁-C₄-alkyl, C₅-C₆-cycloalkyl, (C₆-C₁₂)-aryl-(C₁-C₃)-alkyl or C₆-C₁₂-aryl or -heteroaryl which may be substituted by a hydroxyl, methoxy or trialkylsilyloxy group;
R² is C₆-C₁₂-aryl which may be substituted by a methoxy, halogen, methyl, trifluoromethyl or isopropyl group;
is C₃-C₆-heteroaryl which may be substituted by a methoxy, halogen, methyl, trifluoromethyl or isopropyl group, or
is C₁-C₄-alkyl, -alkenyl or -alkynyl.

3. The process as claimed in claim 1 or 2, wherein
R¹ is C₁-C₄-alkyl,
(C₆-C₁₂)-aryl-(C₁-C₃)-alkyl or C₆-C₁₂-aryl, and
R² = C₆-C₁₂-aryl, C₃-C₆-heteroaryl or C₁-C₄-alkyl, -alkenyl or -alkynyl.

4. The process as claimed in one or more of claims 1 - 3, wherein a compound of the formula IIa or IIb or IIIa or IIIb, respectively, in which
IIa: R³ = Me, R⁵ = Ph, R⁴ = R⁶ = H
IIb: R⁴ = Me, R⁶ = Ph, R³ = R⁵ = H
IIIa: R³ = CH(CH₃)₂, R⁴ = R⁵ = R⁶ = H
IIIb: R⁴ = CH(CH₃)₂, R³ = R⁵ = R⁶ = H
and Y is = O is employed.

5. The process as claimed in one or more of claims 1 - 4, wherein the compound of the formula IIa or IIb or IIIa or IIIb, respectively, is converted in accordance with a₃) in one step directly into a compound of the formula VIIIa or VIIIb or IXa or IXb.

6. The process as claimed in one or more of claims 1 - 5, wherein the alkylation of the compound of the formula VIIIa, VIIIb, IXa or IXb, the subsequent elimination of the chiral auxiliary and the oxidation to give the compounds of the formula XII or I, respectively, is carried out without isolating the intermediates.

## Revendications

1. Procédé pour la préparation stéréosélective d'un composé de formule I 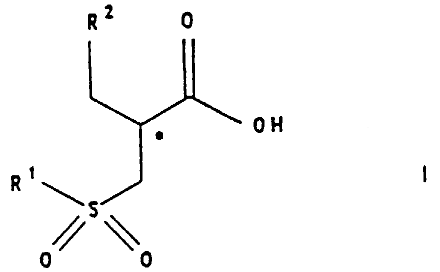 dans laquelle
R¹ représente un groupe alkyle en C₁-C₆, cycloalkyle en C₃-C₆, aryl(C₆-C₁₂)alkyle(C₁-C₄) ou aryle en C₆-C₁₂ ou hétéroaryle en C₆-C₁₂ ou hétérocycloalkyle en C₆-C₁₂, qui peut être substitué par 1, 2 ou 3 groupes hydroxyle, méthoxy ou trialkylsilyloxy, identiques ou différents; et
R² représente un groupe aryle en C₆-C₁₂ qui peut être substitué par 1, 2 ou 3 groupes méthoxy, halogéno, cyano, méthyle, trifluorométhyle, isopropyle ou nitro, identiques ou différents;
un groupe hétéroaryle en C₃-C₉ qui peut être substitué par 1, 2 ou 3 groupes méthoxy, halogéno, cyano, méthyle, trifluorométhyle, isopropyle ou nitro, identiques ou différents; ou
un groupe alkyle, alcényle ou alcynyle en C₁-C₁₀, qui peut être substitué par 1, 2 ou 3 groupes méthoxy, halogéno, cyano, méthyle, trifluorométhyle, isopropyle ou nitro, identiques ou différents,
et les composés de formule I peuvent se trouver sous la forme R ou S,
caractérisé en ce que l'on convertit un composé de formule IIa ou IIb ou IIIa ou IIIb (auxiliaire chiral), où Y = O ou S, 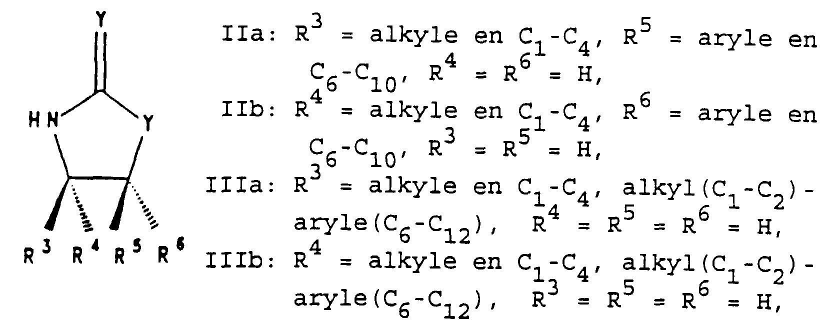
a₁) avec de l'acide acrylique ou des dérivés de l'acide acrylique, en un composé de formule IVa ou IVb ou Va ou Vb, où Y = O ou S, 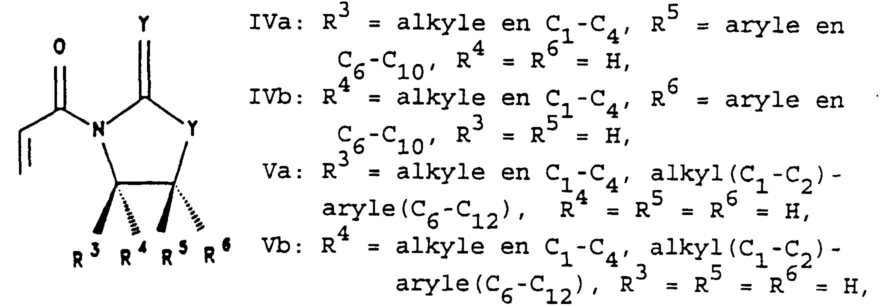 ou
a₂) avec de l'acide propionique ou des dérivés d'acide propionique, qui, pour leur part, sont substitués en position C₃ par un radical X = Cl, Br, OTs, OMs, en un composé de formule VIa ou VIb ou VIIa ou VIIb, où X et Y ont les significations données plus haut 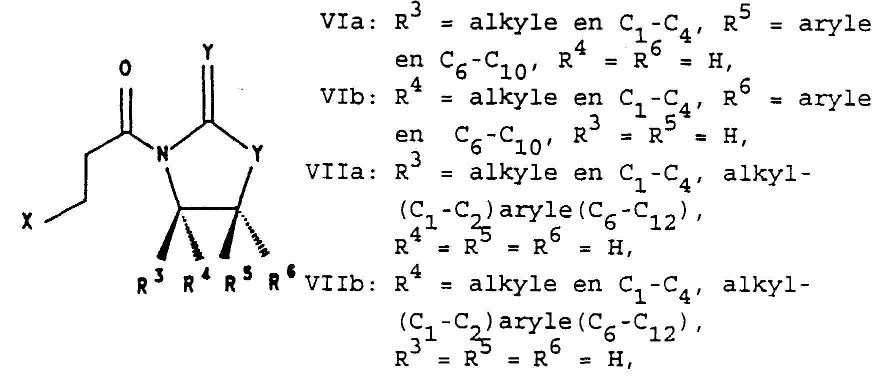
b) on convertit le composé de formule IVa, IVb, Va, Vb, VIa, VIb, VIIa ou VIIb, obtenu selon a₁) ou a₂), directement ou après isolement en milieu non aqueux ou en présence de catalyseurs de transfert de phase, dans des systèmes aqueux biphasiques, avec un mercaptan de formule R¹SH, dans laquelle R¹ est tel que défini plus haut, en le composé correspondant de formule VIIIa ou VIIIb ou IXa ou IXb,
formules dans lesquelles R¹ et Y ont les significations données plus haut, 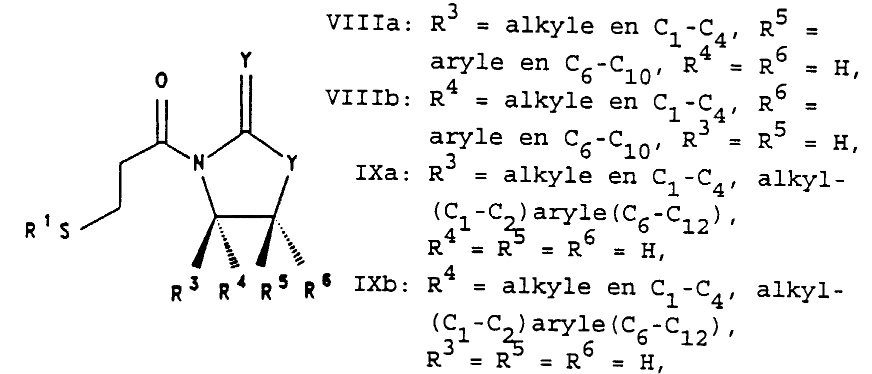 ou
a₃) on convertit un composé de formule IIa ou IIb ou IIIa ou IIIb, en présence d'une quantité de base suffisante pour la déprotonation de l'oxazolidinone ainsi que du mercaptan R¹SH, dans lequel R¹ a la signification donnée plus haut, par la réaction avec des halogénures d'acide acrylique ou propionique ou leurs anhydrides mixtes respectifs, et la réaction ultérieure du produit de réaction avec le mercaptan, en une étape, en un composé de formule VIIIa ou VIIIb ou IXa ou IXb,
c) on convertit ensuite le composé obtenu selon b) ou a₃), par alkylation stéréosélective, en le composé correspondant de formule Xa, Xb, XIa ou XIb, 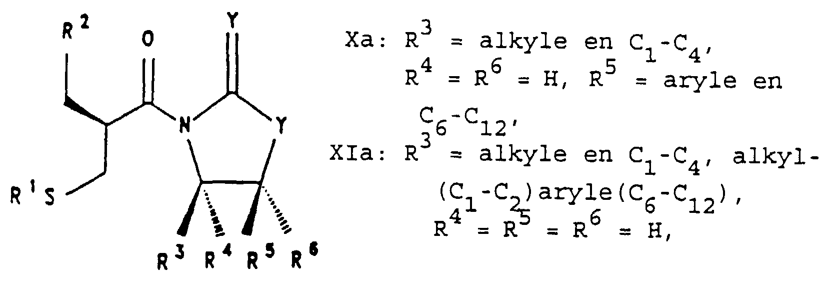 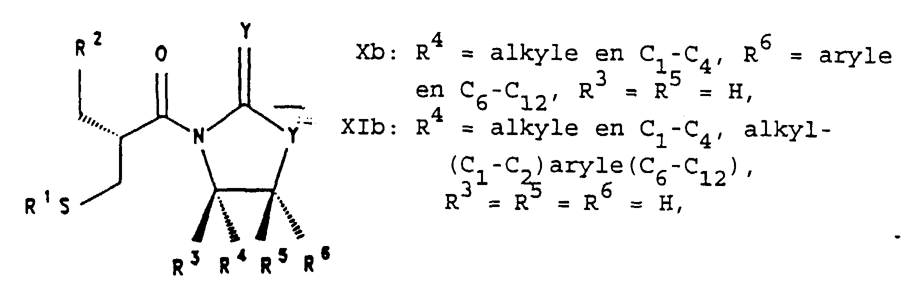 Y, R¹ et R² ayant les significations données plus haut,
d) on convertit ensuite le composé ainsi obtenu, par une réaction avec H₂O₂, en solution alcaline, de préférence avec LiOH/H₂O₂, en composés de formule XII, qui peuvent se trouver sous la forme R ou S, 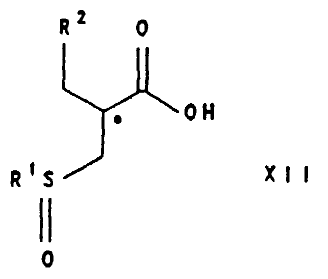 et
e) on convertit ensuite le composé ainsi obtenu, par une nouvelle oxydation, en le composé de formule I correspondant.

2. Procédé selon la revendication 1, caractérisé en ce que
R¹ représente un groupe alkyle en C₁-C₄, cycloalkyle en C₅-C₆, aryl(C₆-C₁₂)alkyle (C₁-C₃) ou aryle en C₆-C₁₂ ou hétéroaryle en C₆-C₁₂, qui peut être substitué par un groupe hydroxyle, méthoxy ou trialkylsilyloxy;
R² représente un groupe aryle en C₆-C₁₂ qui peut être substitué par un atome d'halogène ou par le groupe méthoxy, méthyle, trifluorométhyle ou isopropyle; un groupe hétéroaryle en C₃-C₆ qui peut être substitué par un atome d'halogène ou par le groupe méthoxy, méthyle, trifluorométhyle ou isopropyle, ou un groupe alkyle, alcényle ou alcynyle en C₁-C₄.

3. Procédé selon la revendication 1 ou 2, caractérisé en ce que
R¹ représente un groupe alkyle en C₁-C₄, aryl (C₆-C₁₂) - alkyle(C₁-C₃) ou aryle en C₆-C₁₂, et
R² représente un groupe aryle en C₆-C₁₂, hétéroaryle en C₃-C₆ ou alkyle, alcényle ou alcynyle en C₁-C₄.

4. Procédé selon une ou plusieurs des revendications 1 à 3, caractérisé en ce que l'on utilise un composé de formule IIa ou IIb ou IIIa ou IIIb, formules dans lesquelles
IIa: R³ = Me, R⁵ = Ph, R⁴ = R⁶ = H,
IIb: R⁴ = Me, R⁶ = Ph, R³ = R⁵ = H,
IIIa: R³ = CH(CH₃)₂, R⁴ = R⁵ = R⁶ = H,
IIIb: R⁴ = CH(CH₃)₂, R³ = R⁵ = R⁶ = H,
et Y = O.

5. Procédé selon une ou plusieurs des revendications 1 à 4, caractérisé en ce que l'on convertit le composé de formule IIa ou IIb ou IIIa ou IIIb selon a₃) en une étape, directement en un composé de formule VIIIa ou VIIIb ou IXa ou IXb.

6. Procédé selon une ou plusieurs des revendications 1 à 5, caractérisé en ce que l'on effectue sans isolement des produits intermédiaires l'alkylation du composé de formule VIIIa, VIIIb, IXa ou IXb, la dissociation subséquente du produit auxiliaire chiral ainsi que l'oxydation en les composés de formule XII ou I, respectivement.
